(19) Europäisches Patentamt
European Patent Office
Office européen des brevets

(11) Veröffentlichungsnummer: **0 287 867 A1**

(12) **EUROPÄISCHE PATENTANMELDUNG**

(21) Anmeldenummer: **88105149.4**

(22) Anmeldetag: **30.03.88**

(51) Int. Cl.⁴: **C07D 401/12 , C07D 401/14 , C07D 409/14 , A61K 31/505**

(30) Priorität: **11.04.87 DE 3712369**

(43) Veröffentlichungstag der Anmeldung: **26.10.88 Patentblatt 88/43**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Franckowiak, Gerhard, Dr.**
**Henselweg 10**
**D-5600 Wuppertal 1(DE)**
Erfinder: **Kayser, Michael, Dr.**
**Walter-Flex-Strasse 18**
**D-5090 Leverkusen(DE)**
Erfinder: **Schramm, Matthias, Dr.**
**Paffrather Strasse 38**
**D-5000 Köln 80(DE)**
Erfinder: **Thomas, Günther, Dr.**
**Via Campognallo 21/14**
**I-20020 Arese (Mi)(IT)**
Erfinder: **Gross, Rainer, Prof. Dr.**
**Platzhofstrasse 23**
**D-5600 Wuppertal 1(DE)**
Erfinder: **Perzborn, Elisabeth, Dr.**
**Am tescher Busch 13**
**D-5600 Wuppertal 11(DE)**
Erfinder: **Seuter, Friedel, Dr.**
**Moospfad 16**
**D-5600 Wuppertal 1(DE)**

(54) **Substituierte 5-Nitro-1,4-dihydropyridine, Verfahren zu ihrer Herstellung und ihre Verwendung.**

(57) Die Erfindung betrifft substituierte 5-Nitro-1,4-dihydropyridine der allgemeinen Formel I

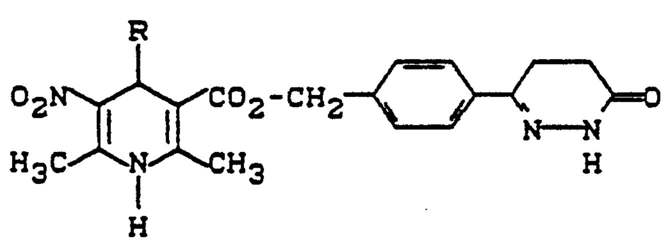

(I),

in welcher R die in der Beschreibung angegebene Bedeutung hat, mehrere Verfahren zu ihrer Herstellung und ihre Verwendung in Arzneimitteln, insbesondere zur Bekämpfung von Kreislauferkrankungen.

EP 0 287 867 A1

## Substituierte 5-Nitro-1,4-dihydropyridine, Verfahren zur Herstellung und ihre Verwendung

Die Erfindung betrifft substituierte 5-Nitro-1,4-dihydropyridine, mehrere Verfahren zu ihrer Herstellung und ihre Verwendung in Arzneimitteln, insbesondere zur Bekämpfung von Kreislauferkrankungen.

Die vorliegende Erfindung betrifft substituiert 5-Nitro-1,4-dihydropyridine der allgemeinen Formel (I)

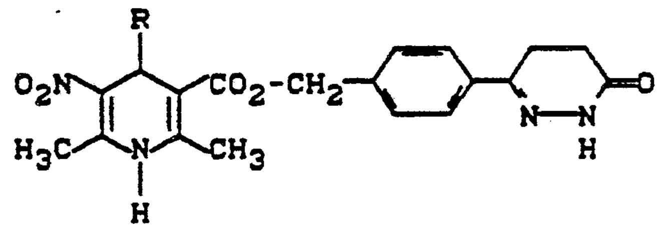

(I),

in welcher

R - für $C_6$-$C_{14}$-Aryl steht, das bis zu fünffach gleich oder verschieden substituiert sein kann durch Halogen, Nitro, Cyano, $C_1$-$C_6$-Alkyl, $C_1$-$C_6$-Alkoxy, $C_1$-$C_6$-Alkylthio, Trifluormethyl, Trifluormethoxy, Difluormethoxy, Trifluormethylthio oder durch gegebenenfalls durch Nitro, Cyano, Trifluormethyl, $C_1$-$C_4$-Alkyl oder $C_1$-$C_4$-Alkoxy substituiertes Benzyl, Benzyloxy oder Benzylthio, oder

- für einen gegebenenfalls bis zu vierfach durch Halogen, Phenyl oder $C_1$-$C_4$-Alkyl substituierten Heterocyclus aus der Reihe Pyrryl, Thienyl, Furyl, Pyrazolyl, Imidazolyl, Oxazolyl, Thiazolyl, Pyridyl, Pyridazinyl, Pyrimidyl, Chinolyl, Benzoxadiazolyl, Chromenyl oder Thiochromenyl steht, oder

- für geradkettiges, verzweigtes oder cyclisches Alkyl mit bis zu 14 Kohlenstoffatomen steht,

und deren physiologisch unbedenkliche Salze.

Bevorzugt sind Verbindungen der allgemeinen Formel (I),

in welcher

R - für Phenyl oder Naphthyl steht, das bis zu vierfach gleich oder verschieden substituiert sein kann durch Fluor, Chlor, Brom, Nitro, Cyano, $C_1$-$C_4$-Alkyl, $C_1$-$C_4$-Alkoxy, $C_1$-$C_4$-Alkylthio, Trifluormethyl, Difluormethoxy, Trifluormethoxy, Trifluormethylthio, durch gegebenenfalls durch Nitro, Fluor, Chlor, Brom, Trifluormethyl, Methyl oder Methoxy substituiertes Benzyloxy oder Benzylthio, oder

- für einen gegebenenfalls bis zu dreifach durch Methyl, Fluor, Chlor, Brom oder Phenyl substituierten Heterocyclus aus der Reihe Thienyl, Furyl, Pyridyl, Pyrimidyl, Chinolyl, Benzoxadiazolyl oder Thiochromenyl steht, oder

- für geradkettiges, verzweigtes oder cyclisches Alkyl mit bis zu 12 Kohlenstoffatomen steht,

und deren physiologisch unbedenkliche Salze.

Besonders bevorzugt sind Verbindungen der allgemeinen Formel (I),

in welcher

R - für Phenyl steht, das bis zu dreifach gleich oder verschieden substituiert sein kann durch Fluor, Chlor, Nitro, Cyano, $C_1$-$C_4$-Alkyl, $C_1$-$C_4$-Alkoxy, Trifluormethyl, Trifluormethoxy, durch gegebenenfalls durch Nitro, Fluor, Chlor, Brom, Trifluormethyl, Methyl oder Methoxy substituiertes Benzyloxy oder Benzylthio, oder

- für Thienyl, Furyl, Pyridyl, Benzoxadiazolyl oder

- für einen Heterocyclus der Formel

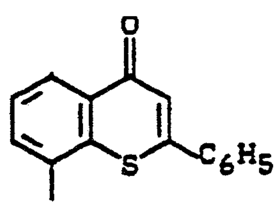

steht, oder

- für cyclisches Alkyl mit bis zu 10 C-Atomen wie Cyclohexyl oder Adamantyl steht,

und deren physiologisch unbedenkliche Salze.

Physiologisch unbedenkliche Salze sind Salze der erfindungsgemäßen Verbindungen mit anorganischen oder organischen Säuren. Hierzu gehören bevorzugt anorganische Säuren wie Halogenwasserstoffsäuren, bevorzugt HCl oder HBr, Schwefelsäure, Phosphorsäure, oder organische Carbonsäuren oder

Sulfonsäuren wie beispielsweise Essigsäure, Maleinsäure, Fumarsäure, Äpfelsäure, Citronensäure, Weinsäure, Milchsäure, Benzoesäure, Methansulfonsäure, Ethansulfonsäure, Benzolsulfonsäure, Naphthalindisulfonsäure oder Toluolsulfonsäure.

Die erfindungsgemäßen Verbindungen existieren in stereoisomeren Formen, die sich entweder wie Bild und Spiegelbild (Enantiomere) oder die sich nicht wie Bild und Spiegelbild (Dastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Verbindungen der allgemeinen Formel (I) werden hergstellt, indem man

[A] Aldehyde der allgemeinen Formel (II)

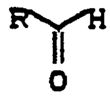 (II)

in welcher
R die angegebene Bedeutung hat,
mit Nitroaceton der Formel (III),

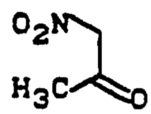 (III)

und dem Enamin der Formel (IV)

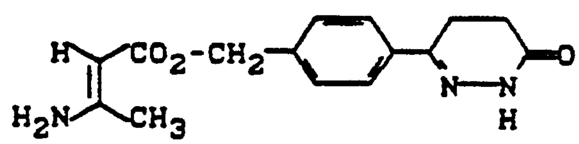 (IV)

in Gegenwart inerter Lösemittel umsetzt,
oder indem man

[B] Aldehyde der allgemeinen Formel (II), mit dem Keton der Formel (V),

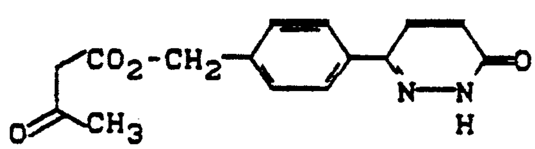 (V)

und dem Enamin der Formel (VI)

VI 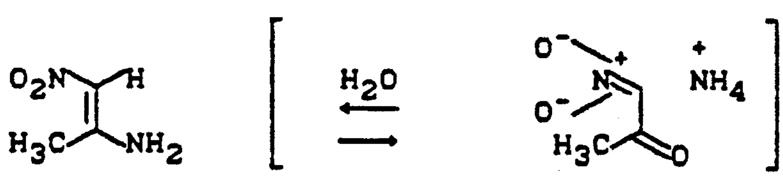


in Gegenwart inerter Lösemittel umsetzt,
oder indem man

[C] Nitroaceton (III) mit Ammoniak und Ylidenverbindungen der allgemeinen Formel (VII)

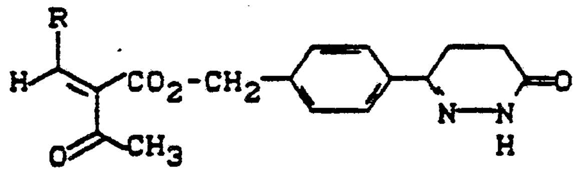

(VII)

in welcher
R die angegebene Bedeutung hat,
in Gegenwart inerter Lösemittel umsetzt,
oder indem man

[D] das Keton der Formel (V), mit Ammoniak und Ylidenverbindungen der allgemeinen Formel (VIII)

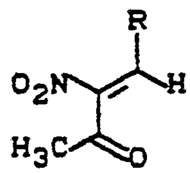

(VIII)

in welcher
R die angegebene Bedeutung hat,
in Gegenwart inerter Lösemittel umsetzt,
oder indem man

[E] das Enamin der Formel (VI) mit Ylidenverbindungen der allgemeinen Formel (VII) in Gegenwart inerter Lösemittel umsetzt.
oder indem man

[F] das Enamin der Formel (IV) mit Ylidenverbindungen der allgemeinen Formel (VII) in Gegenwart inerter Lösemittel umsetzt,
oder indem man

[G] 1,4-Dihydropyridincarbonsäuren der allgemeinen Formel (X)

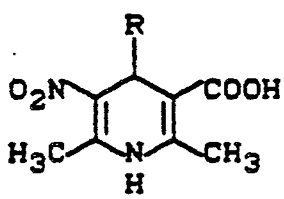

(X)

in welcher
R die angegebene Bedeutung hat,
nach üblichen Methoden der Veresterung von Carbonsäuren (z.B. über das Säurechlorid oder -imidazolid oder in Gegenwart von Dicylcohexylcarbodiimid) mit dem Alkohol der Formel (XI)

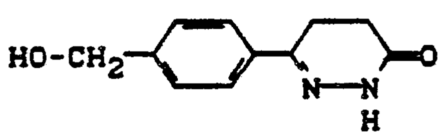

(XI)

verestert.

Je nach Art der eingesetzten Ausgangsverbindungen lassen sich die Verfahrensvarianten A - F durch folgende Schemata verdeutlichen:

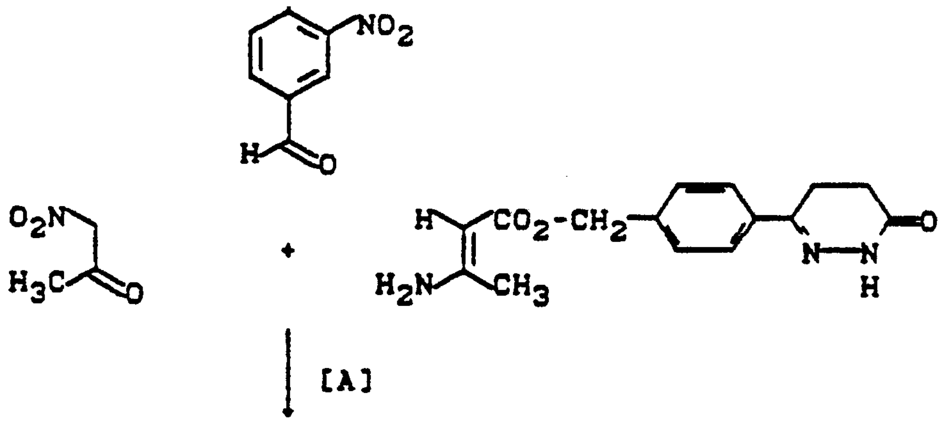
[A]

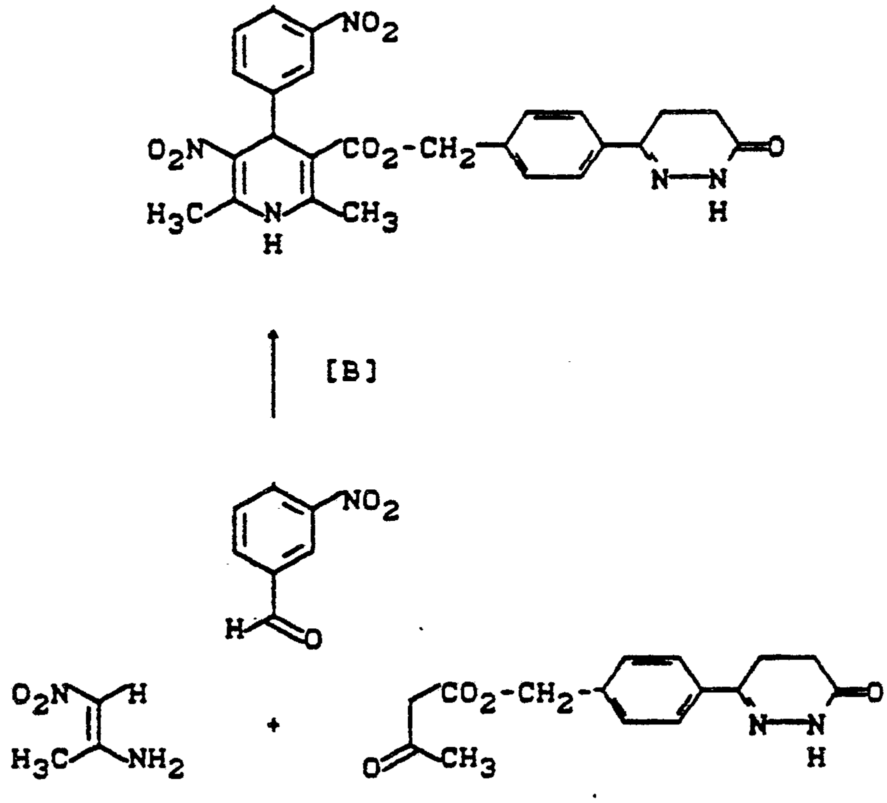
[B]

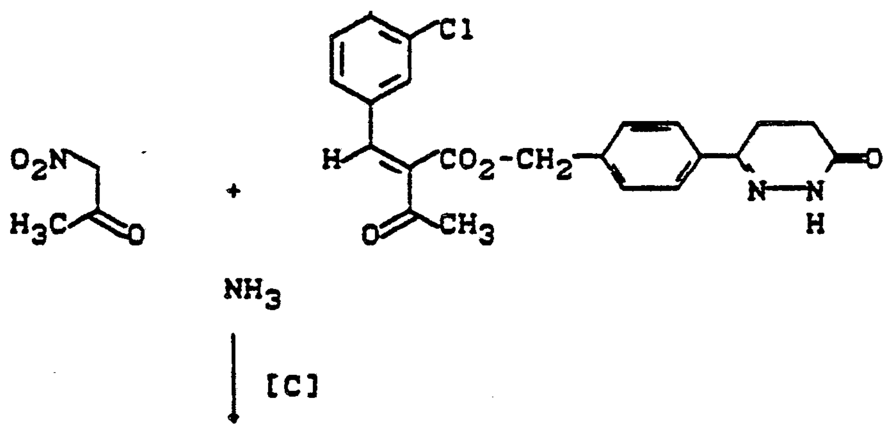
NH3
[C]

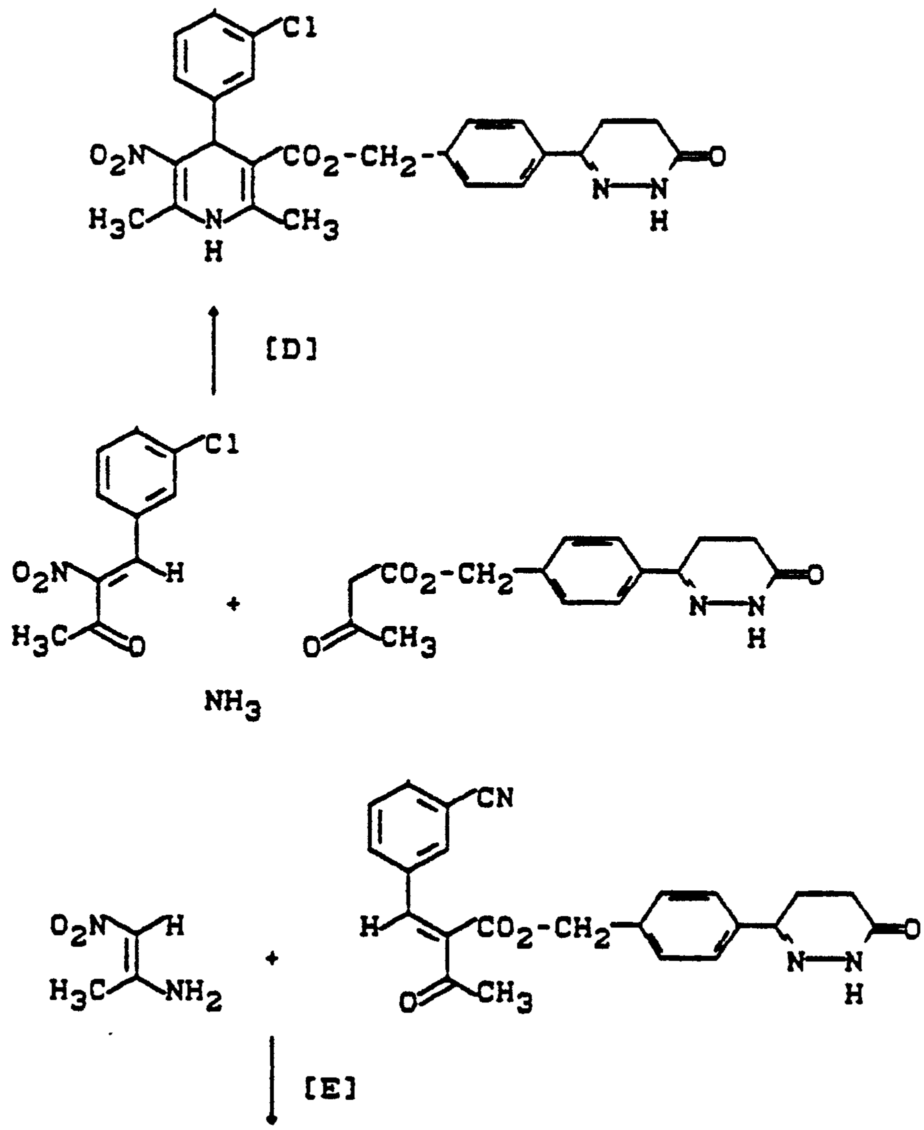
[D]
NH3
[E]

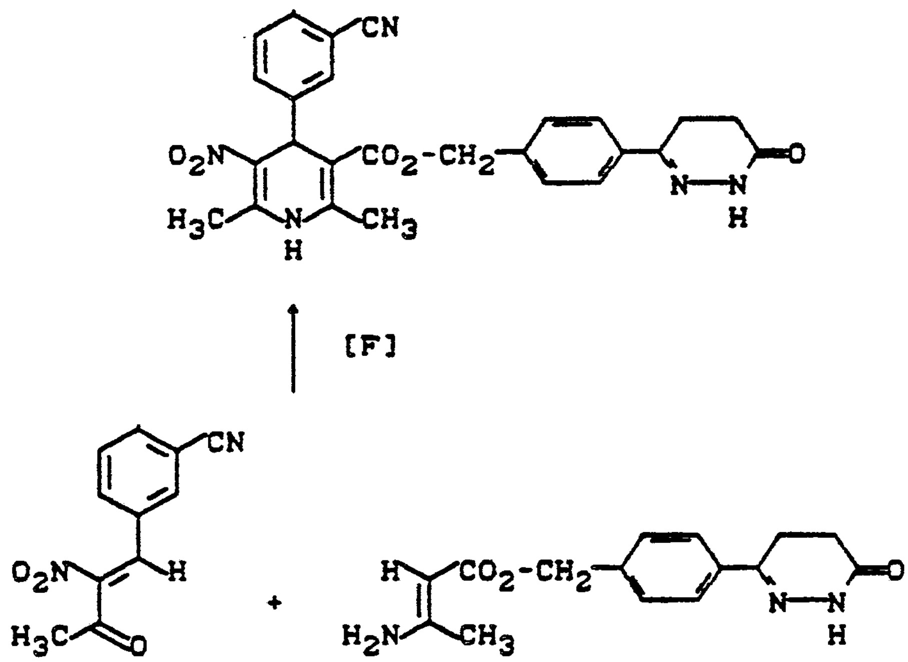


[G]

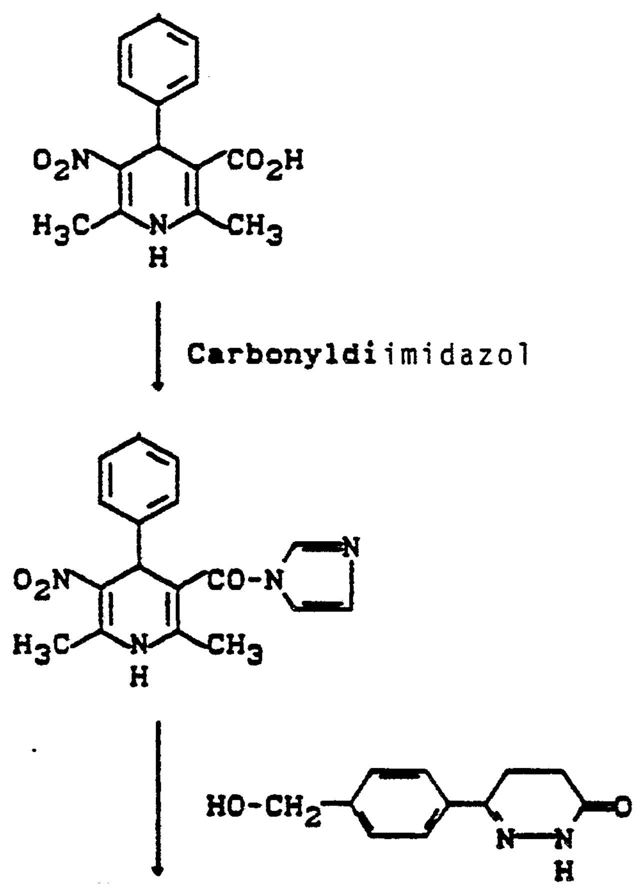

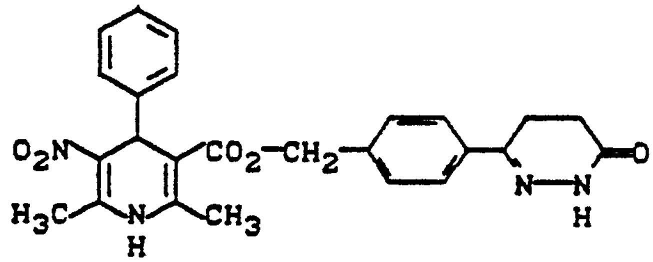

Die als Ausgangsstoffe eingesetzten Aldehyde der allgemeinen Formel (II) sind bekannt oder können nach bekannten Methoden hergestellt werden [E. Mosettig, Organic Reactions III, 218, (1954); E.P. Papadopoulos, A. Jarrar, C.H. Isidorides, J. Org. Chem. 31, 615 (1966); Mijano et al., Chemical Abstracts 59, 13 929 (1963); A.J. Mancuso, D.S. Brownfain, D. Swern, J. Org. Chem. 44, 4148 (1979); T.D. Harris, G.P. Roth, J. Orgn Chem. 44, 2004 (1979)].

Das Nitroaceton (III) ist bekannt und kann nach bekannten Methoden hergestellt werden [N. Levy, C.W. Scaife, J. Chem. Soc. 1949, 1100; C.D. Hurd, M.E. Nilson, J. Org. Chem. 20, 927 (1955), G.F. Field, W.J. Zally, Synthesis 295 (1979)].

Das als Ausgangsstoff eingesetzte Keton der Formel (V) kann nach bekannten Methoden hergestellt werden [D. Borrman, in Houben-Weyls "Methoden der organischen Chemie" VII/4, 230 (1968)].

Die als Ausgangsstoffe eingesetzten Enamine der Formeln (IV) und (VI) sind bekannt oder können nach bekannten Methoden hergestellt werden [S.A. Glickman, A.C. Cope, J. Am. Chem. Soc. 67, 1017 (1945)); H. Böhme, K.-H. Waisel, Arch. Pharm. 310, 30 (1977)].

Die als Ausgangsstoffe eingesetzten Ylidenverbindungen der allgemeinen Formeln (VII) und (VIII) sind teilweise bekannt oder können nach bekannten Methoden hergestellt werden [G. Jones, Organic Reactions XV, 204 (1967); A. Dornow, W. Sassenberg, Liebigs. Am. Chem. 602 14 (1957)].

Die als Ausgangsstoffe eingesetzten Carbonsäuren der Formel (X) sind bekannt oder können nach bekannten Methoden hergestellt werden [DE 32.06.671].

Verfahrensvarianten A - F

Als Lösemittel für Verfahren A - F können Wasser und alle inerten organischen Lösemittel verwendet werden, die sich unter den Reaktionsbedingungen nicht verändern. Hierzu gehören bevorzugt Alkohole wie Methanol, Ethanol, Propanol, Isopropanol, Ether wie Diethylether, Dioxan oder Tetrahydrofuran, Dimethylformamid, Dimethylsulfoxid, Acetonitril, Eisessig, Essigester, Hexamethylphosphorsäuretriamid, oder Kohlenwasserstoffe wie Benzol, Toluol oder Xylol. Ebenso können Gemische der genannten Lösemittel eingesetzt werden.

Die Reaktionstemperaturen für alle Verfahren können in einem größeren Bereich variiert werden. Im allgemeinen arbeitet man in einem Bereich von +10°C bis +200°C, bevorzugt von +20°C bis 150°C, insbesonders bei der Siedetemperatur des verwendeten Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung des erfindungsgemäßen Verfahrens ist das Verhältnis der an der Reaktion beteiligten Stoffe beliebig. Im allgemeinen arbeitet man mit molaren Mengen der Reaktanden. Bei Verfahren C und D hat es sich als zweckmäßig erwiesen, Ammoniak im bis zu 200fachen, bevorzugt bis zu 10fachen Überschuß einzusetzen.

Verfahrensvariante G

Die Durchführung der erfindungsgemäßen Verfahrensvariante G lehnt sich an die literaturbekannte Methodik zur Veresterung von Carbonsäuren an. Dabei wird die Carbonsäure zunächst in eine aktivierte Form wie z.B. das Säurechlorid oder das Imidazolid überführt, die entweder als solche isoliert und in einem zweiten Reaktionsschritt umgesetzt wird oder die in situ direkt zu den erfindungsgemäßen Verbindungen alkanolisiert wird. Als aktivierende Agentien seien neben den anorganischen Halogeniden wie Thionylchlorid,

Phosphortrichlorid oder Phosphorpentachlorid auch Carbonyldiimidazol, Carbodiimide wie Dicyclohexylcarbodiimid oder 1-Cyclohexyl-3-[2-(N-methyl-morpholino)ethyl]carbodiimid-p-toluolsulfonat oder N-Hydroxyphthalimid oder N-Hydroxy-benzotriazol in Gegenwart von Dicyclohexylcarbodiimid bei spielhaft erwähnt. Naturgemäß lassen sich die 1,4-Dihydropyridinmonocarbonsäuren auch in Salze überführen, die mit Substraten der allgemeinen Formel (XII)

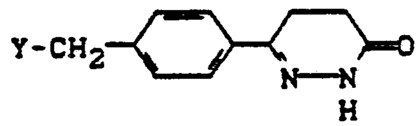

(XII)

in welcher
Y - eine nucleofuge Gruppe wie beispielsweise Iodid oder Tosylat darstellt,
zu den erfindungsgemäßen Verbindungen umgesetzt werden können.

Als Verdünnungsmittel kommen alle inerten organischen Lösemittel in Frage. Hierzu gehören vorzugsweise Ether wie Dioxan, Tetrahydrofuran, Glykolmonomethylether oder Glykoldimethylether, halogenierte Kohlenwasserstoffe wie Dichlormethan oder Trichlormethan, Dimethylformamid, Dimethylsulfoxid, Acetonitril, Pyridin und Hexamethylphosphorsäuretriamid. Isoliert man die aktivierten Zwischenstufen der 1,4-Dihydromonocarbonsäuren, so können der Alkohol der Formel (XI) auch allein als Verdünnungsmittel eingesetzt werden.

Die Alkanolyse wird zweckmäßigerweise durch Zugabe katalytischer oder molarer Mengen eines basischen Hilfsstoffs beschleunigt.

Die Reaktionstemperaturen können in einem größeren Bereich variiert werden. In allgemeinen arbeitet man zwischen +10°C und +200°C, insbesondere zwischen +20°C und +150°C, vorzugsweise jedoch bei der Siedetemperatur des jeweiligen Lösemittels.

Die Umsetzung kann bei Normaldruck, aber auch bei erhöhtem Druck durchgeführt werden. Im allgemeinen arbeitet man unter Normaldruck.

Die erfindungsgemäßen Verbindungen beeinflussen die Kontraktionskraft des Herzens und den Tonus der glatten Muskulatur. Sie können deshalb in Arzneimitteln zur Beeinflussung des pathologisch veränderten Blutdrucks, als Koronartherapeutika und zur Behandlung der Herzinsuffizienz, eingesetzt werden. Darüber hinaus können sie zur Behandlung von Herzrhythmusstörungen, zur Senkung des Blutzuckers, zur Abschwellung von Schleimhäuten und zur Beeinflussung des Salz-und Flüssigkeitshaushaltes verwendet werden.

Die Herz-und Gefäßwirkungen wurden am isoliert perfundierten Herzen des Meerschweinchens gefunden.

Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0.013 mmol/l $Na_2EDTA$), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32°C mittels einer Rollenquetschpumpe perfundiert.

Zur Messung der Herzfunktion wird ein flüssigkeitsgefüllter Latexballon, der über eine Flüssigkeitssäule miteinem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965) 529 - 541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Zu-bzw. Abnahme der linksventrikulären Kontraktionsamplitude einen Anstieg bzw. eine Senkung der Herzkontraktilität an. Die erfindungsgemäßen Verbindungen werden in geeigneten Verdünnungen in das Perfusionsmedium kurz vor dem isolierten Herzen infundiert.

Die folgenden Werte zeigen beispielhaft den Effekt der erfindungsgemäßen Verbindungen am isoliert perfundierten Meerschweinchenherzen, ausgedrückt als prozentuale Differenz gegenüber dem gleich 1000% gesetzten Ausgangswert.

| Bsp.-Nr. | Konzentration (g/ml) | %-Änderung der Kontraktionskraft | %-Änderung des Perfusionsdruckes |
|---|---|---|---|
| 2 | $10^{-6}$ | +22 | -35 |
| 3 | $10^{-6}$ | +6 | 0 |
| 4 | $10^{-6}$ | +63 | -35 |
| 5 | $10^{-6}$ | +102 | -45 |
| 6 | $10^{-6}$ | +48 | -11 |
| 7 | $10^{-6}$ | +56 | -22 |
| 8 | $10^{-6}$ | +5 | -21 |
| 9 | $10^{-6}$ | +16 | -10 |
| 11 | $10^{-6}$ | +35 | -37 |
| 12 | $10^{-6}$ | +70 | -25 |
| 13 | $10^{-6}$ | +6 | -10 |
| 14 | $10^{-6}$ | +15 | -6 |
| 15 | $10^{-6}$ | +22 | -14 |
| 18 | $10^{-6}$ | +46 | 0 |
| 22 | $10^{-6}$ | +78 | -10 |
| 23 | $10^{-6}$ | +40 | 0 |

| Bsp.-Nr. | Konzentration (g/ml) | %-Änderung der Kontraktionskraft | %-Änderung des Perfusionsdruckes |
|---|---|---|---|
| 25 | $10^{-6}$ | +72 | 0 |
| 27 | $10^{-6}$ | +28 | -10 |
| 28 | $10^{-6}$ | +9 | -16 |

Darüberhinaus wirken die erfindungsgemäßen Stoffe der Formel (I) als Hemmer/Stimulatoren von enzymatischen Reaktionen im Rahmen des Arachidonsäurestoffwechsels. Derartige Substanzen sind zur Verhütung und Behandlung von Erkrankungen der Atemwege wie Emphysen, Schocklunge, pulmonaler Hypertonie, Ödem, Thrombose und Thromboembolie, Ischämien (periphere, coronare, cerebrale Durchblutungsstörungen), Herz-und Hirninfarkte, Herzrhythmusstörungen, Angina pectoris, Hypertonie sowie Arteriosklerose geeignet.
Die erfindungsgemäßen Stoffe wirken bevorzugt thrombozytenaggregationshemmend.

Thrombozytenaggregation

Thrombozyten und deren Adhäsion - sowie Aggregationsfähigkeit - sind, besonders im arteriellen Schenkel des Gefäßsystems, ein wesentlicher pathogenetischer Faktor bei der Entstehung von Thrombosen.

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt, Mittels Zentrifugation erhält man aus diesem Blut plättchenreiches Zitratplasma (PRP) (Jürgen/Beller, Klinische Methoden der Blutgerinnungsanalyse; Thieme Verlag, Stuttgart, 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode (Born, G.V.R., J. Physiol. (London), 162, 67, 1962) im Aggregometer bei 37°C bestimmt (Therapeutische Berichte 47, 80 - 86, 1975). Hierzu wurde die vorinkubierte Probe mit 0,1 ml Kollagen, einem aggregationsauslösenden Agens, versetzt. Die Veränderung der optischen Dichte in der Probe des PRP wurde während einer Zeitdauer von 4 Minuten aufgezeichnet und der Ausschlag nach 4 Minuten bestimmt. Hierzu wird die prozentuale Hemmung gegenüber der Kontrolle errechnet.
Als Grenzkonzentration wird der Bereich der minimal effektiven Konzentrationen angegeben (Tabelle 2).

Kollagen-induzierte Thrombozytenaggregation

| Beispiel-Nr. | Grenzkonzentrationen (µg/ml) |
|---|---|
| 3 | 1 - 0,3 |
| 6 | 10 - 3 |
| 7 | 3 - 1 |
| 8 | 3 - 1 |
| 9 | 3 - 1 |
| 11 | 3 - 1 |
| 12 | 3 - 1 |
| 14 | 1 - 0,3 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Akohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch-und Traubenzucker), Emulgiermittel (z.B. Polyoxyethylen-Fettsäure-Ester, Polyoxyethylen-Fettalkohol-Ether, Alkylsulfonate and Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlag stoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0.001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

1,4-Dihydro-2,6-dimethyl-4-(4-nitrophenyl)-5-nitro-pyridin-3-carbonsäure-4-(6-oxo-1,4,5,6-tetrahydropyridazin-3-yl)benzylester

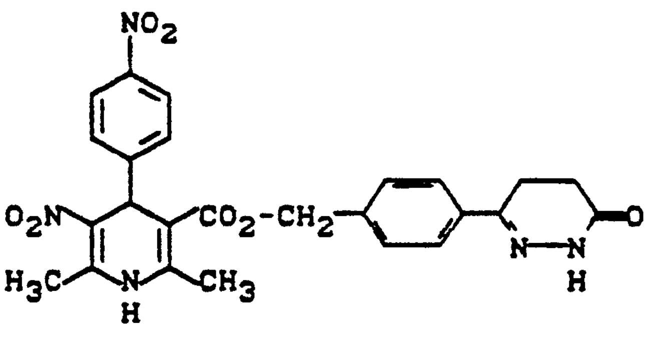

20 mmol 2-Nitro-1-(2-trifluormethylphenyl)-butan-3-on werden mit 20 mmol $\beta$-Aminocrotonsäure-4-(6-oxo-1,4,5,6-tetrahydropyridazin-3-yl)benzylester in 40 ml i-Propanol 6 h zum Rückfluß erhitzt. Nach teilweisem Abdampfen des Lösemittels fällt das Produkt kristallin aus.
Schmp.: 224°C
Ausbeute: 64% der Theorie

Beispiel 2

4-(3-Benzyloxyphenyl)-1,4-dihydro-2,6-dimethyl-5-nitropyridin-3-carbonsäure-4-(6-oxo-1,4,5,6-tetrahydropyridazin-3-yl)benzylester

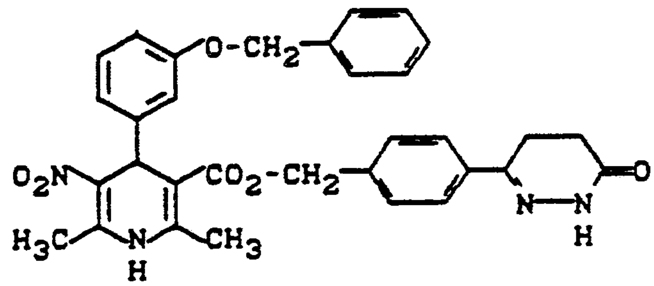

10 mmol 3-Benzyloxybenzaldehyd and 10 mmol β-Aminocrotonsäure-4-(6-oxo-1,4,5,6-tetrahydropyridazin-3-yl)benzylester werden in 30 ml i-Propanol 8 h auf 60°C erhitzt und dabei in Portionen mit insgesamt 20 mmol Nitroaceton versetzt. Das Produkt kristallisiert nach Zugabe von etwas Petrolether und Anreiben. Umkristallisation an wenig i-Propanol.
Schmp.: 134°C
Ausbeute: 42% der Theorie

Beispiel 3

4-(3-Chlorphenyl)-1,4-dihydro-2,6-dimethyl-5-nitropyridin-3-carbonsäure-4-(6-oxo-1,4,5,6-tetrahydropyridazin-3-yl)benzylester

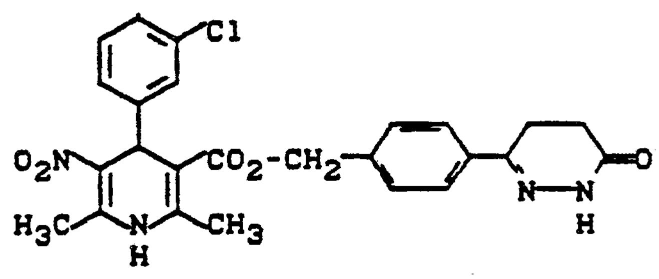

10 mmol 4-(3-Chlorphenyl)-1,4-dihydro-2,6-dimethyl-5-nitro-pyridin-3-carbonsäure werden in 40 ml absolutem Tetrahydrofuran suspendiert und mit 12,5 mmol Carbonyldiimidazol versetzt. Es wird 30 min bei Raumtemperatur und 30 min bei Rückflußtemperatur gerührt. Dann gibt man eine Lösung von 11 mmol 4-(6-Oxo-1,4,5,6-tetrahydropyridazin-3-yl)benzylalkohol und 10 mg Natriummethylat in 10 ml Tetrahydrofuran hinzu und erhitzt 3,5 Stdn. zum Rückflüß. Man engt ein, nimmt den Rückstand in Methylenchlorid auf, wäscht nacheinander mit 1 n Salzsäure, 1 n Natronlauge und Wasser, trocknet mit Magnesiumsulfat und engt ein. Der Rückstand kristallisiert an wenig i-Propanol.
Schmp.: 245°C
Ausbeute: 81% der Theorie

Analog den vorangegangenen Beispielen wurden die folgenden erfindungsgemäßen Verbindungen der Tabelle erhalten:

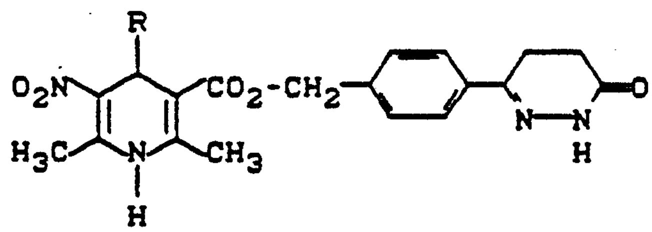

| Nr. | R | Schmp. [°C] |
|---|---|---|
| 4 | 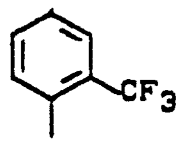 | 233 |

| Nr. | R | Schmp. [°C] |
|---|---|---|
| 5 | 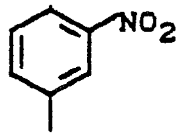 | 270 |
| 6 | 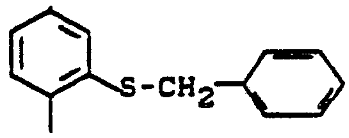 | 140 |
| 7 | 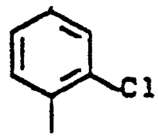 | 264 |
| 8 | 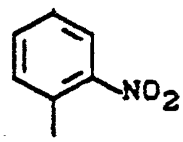 | 254 |
| 9 | 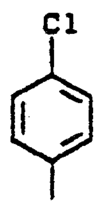 | 234 |
| 10 | 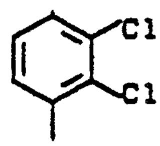 | 257 |
| 11 | 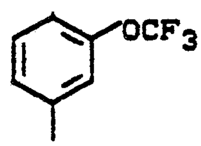 | 224 |
| 12 | 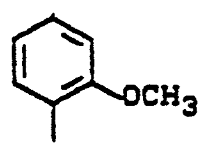 | 264 |

| Nr. | R | Schmp. [°C] |
|---|---|---|
| 13 | 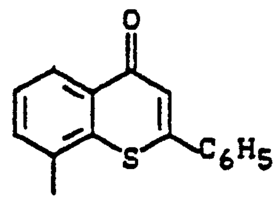 | 257 |
| 14 | 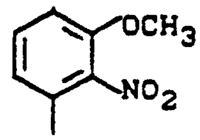 | 146 |
| 15 | 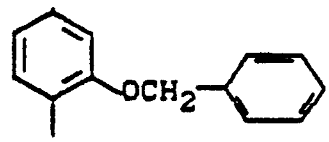 | 126 |
| 16 | 1-Adamantyl | Harz |
| 17 | 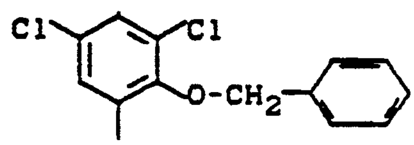 | 198 |
| 18 | 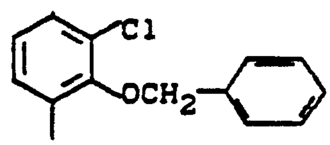 | 138 |
| 19 | 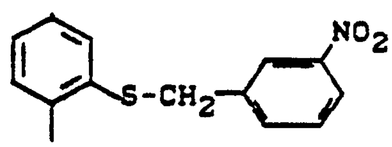 | 126 |
| 20 | 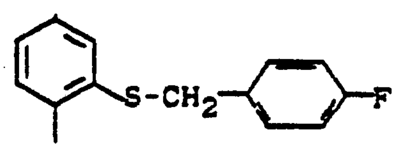 | 145 |

| Nr. | R | Schmp. [°C] |
|---|---|---|
| 21 | 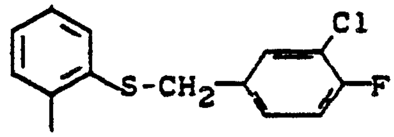 | 148 |
| 22 | 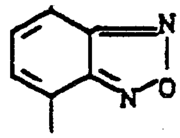 | 230 |
| 23 | 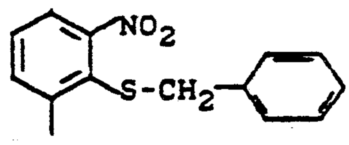 | Harz |
| 24 | 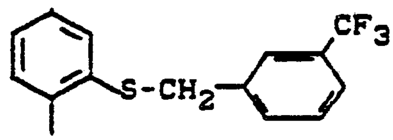 | Harz |
| 25 | 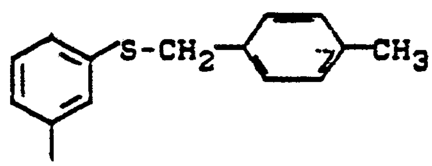 | 130 |
| 26 | 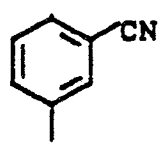 | 273 |
| 27 | 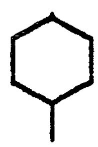 | 148 |
| 28 | 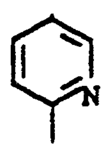 | 159 |
| 29 | 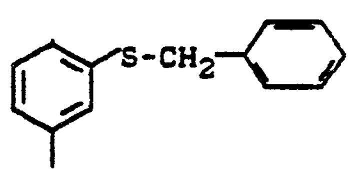 | 184 |

## Ansprüche

1. 5-Nitro-1,4-dihydropyridine der allgemeinen Formel (I)

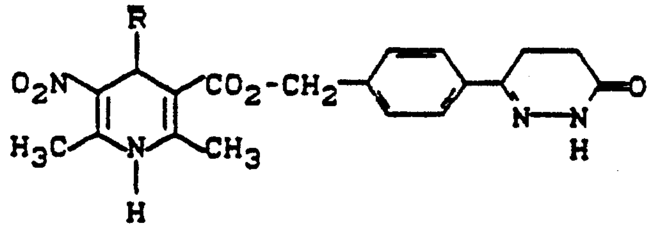

(I),

in welcher
R - für $C_6$-$C_{14}$-Aryl steht, das bis zu fünffach gleich oder verschieden substituiert sein kann durch Halogen, Nitro, Cyano, $C_1$-$C_6$-Alkyl, $C_1$-$C_6$-Alkoxy, $C_1$-$C_6$-Alkylthio, Trifluormethyl, Trifluormethoxy, Difluormethoxy, Trifluormethylthio oder durch gegebenenfalls durch Nitro, Cyano, Trifluormethyl, $C_1$-$C_4$-Alkyl oder $C_1$-$C_4$-Alkoxy substituiertes Benzyl, Benzyloxy oder Benzylthio, oder
- für einen gegebenenfalls bis zu vierfach durch Halogen, Phenyl oder $C_1$-$C_4$-Alkyl substituierten Heterocyclus aus der Reihe Pyrryl, Thienyl, Furyl, Pyrazolyl, Imidazolyl, Oxazolyl, Thiazolyl, Pyridyl, Pyridazinyl, Pyrimidyl, Chinolyl, Benzoxamiazolyl, Chromenyl oder Thiochromenyl steht, oder
- für geradkettiges, verzweigtes oder cyclisches Alkyl mit bis zu 14 Kohlenstoffatomen steht,
und deren physiologisch unbedenkliche Salze.

2. Verbindungen der allgemeinen Formel (I) gemäß Anspruch 1, in welcher
R - für Phenyl oder Naphthyl steht, das bis zu vierfach gleich oder verschieden substituiert sein kann durch Fluor, Chlor, Brom, Nitro, Cyano, $C_1$-$C_4$-Alkyl, $C_1$-$C_4$-Alkoxy, $C_1$-$C_4$-Alkylthio, Trifluormethyl, Trifluormethoxy, Difluormethoxy, Trifluormethylthio, durch gegebenenfalls durch Nitro, Fluor, Chlor, Brom, Trifluormethyl, Methyl oder Methoxy substituiertes Benzyloxy oder Benzylthio,
oder
- für einen gegebenenfalls bis zu dreifach durch Methyl, Fluor, Chlor, Brom oder Phenyl substituierten Heterocyclus aus der Reihe Thienyl, Furyl, Pyridyl, Pyrimidyl, Chinolyl, Benzoxadiazolyl oder Thiochromenyl steht,
oder
- für geradkettiges, verzweigtes oder cyclisches Alkyl mit bis zu 12 Kohlenstoffatomen steht,
und deren physiologisch unbedenkliche Salze.

3. Verbindungen der allgemeinen Formel (I) gemäß Anspruch 1, in welcher
R - für Phenyl steht, das bis zu dreifach gleich oder verschieden substituiert sein kann durch Fluor, Chlor, Nitro, Cyano, $C_1$-$C_4$-Alkyl, $C_1$-$C_4$-Alkoxy, Trifluormethyl, Trifluormethoxy, durch gegebenenfalls durch Nitro, Fluor, Chlor, Brom, Trifluormethyl, Methyl oder Methoxy substituiertes Benzyloxy oder Benzylthio, oder
- für Thienyl, Furyl, Pyridyl, Benzoxadiazolyl oder
- für einen Heterocyclus der Formel

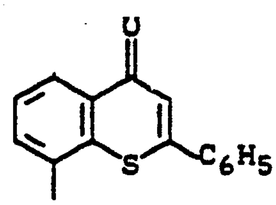

steht, oder
- für cyclisches Alkyl mit bis zu 10 C-Atomen wie Cyclohexyl oder Adamantyl steht,
und deren physiologisch unbedenkliche Salze.

4. Verfahren zur Herstellung von 5-Nitro-1,4-dihydropyridinen der allgemeinen Formel (I)

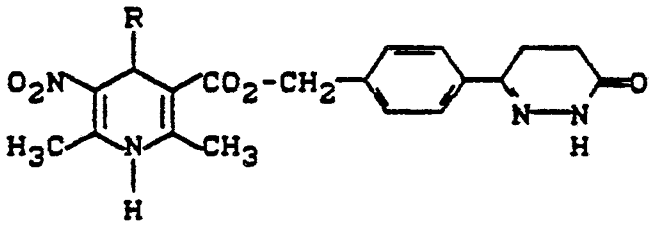

(I),

in welcher

R - für $C_6$-$C_{14}$-Aryl steht, das bis zu fünffach gleich oder verschieden substituiert sein kann durch Halogen, Nitro, Cyano, $C_1$-$C_6$-Alkyl, $C_1$-$C_6$-Alkoxy, $C_1$-$C_6$-Alkylthio, Trifluormethyl, Trifluormethoxy, Difluormethoxy, Trifluormethylthio oder durch gegebenenfalls durch Nitro, Cyano, Trifluormethyl, $C_1$-$C_4$-Alkyl oder $C_1$-$C_4$-Alkoxy substituiertes Benzyl, Benzyloxy oder Benzylthio, oder

- für einen gegebenenfalls bis zu vierfach durch Halogen, Phenyl oder $C_1$-$C_4$-Alkyl substituierten Heterocyclus aus der Reihe Pyrryl, Thienyl, Furyl, Pyrazolyl, Imidazolyl, Oxazolyl, Thiazolyl, Pyridyl, Pyridazinyl, Pyrimidyl, Chinolyl, Benzoxadiazolyl, Chromenyl oder Thiochromenyl steht, oder

- für geradkettiges, verzweigtes oder cyclisches Alkyl mit bis zu 14 Kohlenstoffatomen steht,

und deren physiologisch unbedenklichen Salze,

dadurch gekennzeichnet, daß mit

[A] Aldehyde der allgemeinen Formel (II)

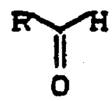

(II)

in welcher

R die angegebene Bedeutung hat,

mit Nitroaceton der Formel (III),

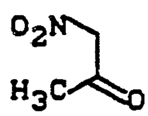

(III)

und dem Enamin der Formel (IV)

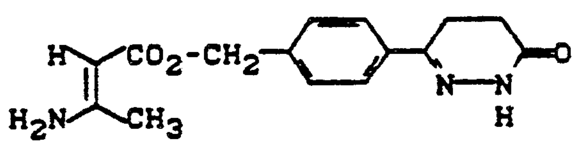

(IV)

in Gegenwart inerter Lösemittel umsetzt,

oder daß man

[B] Aldehyde der allgemeinen Formel (II), mit dem Keton der Formel (V),

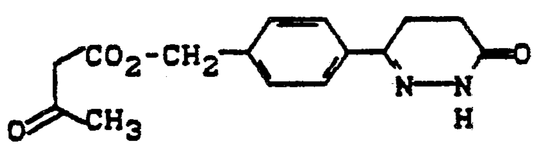

(V)

und dem Enamin der Formel (VI)

VI 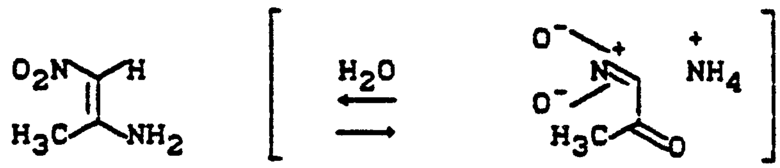


in Gegenwart inerter Lösemittel umsetzt,
oder daß man

[C] Nitroaceton (III) mit Ammoniak und Ylidenverbindungen der allgemeinen Formel (VII)

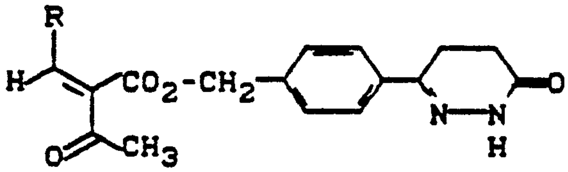 (VII)

in welcher
R die angegebene Bedeutung hat,
in Gegenwart inerter Lösemittel umsetzt,
oder daß man

[D] das Keton der Formel (V), mit Ammoniak und Ylidenverbindungen der allgemeinen Formel (VIII)

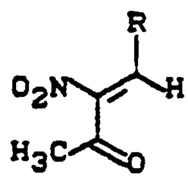 (VIII)

in welcher
R die angegebene Bedeutung hat,
in Gegenwart inerter Lösemittel umsetzt,
oder daß man

[E] das Enamin der Formel (VI) mit Ylidenverbindungen der allgemeinen Formel (VII) in Gegenwart inerter Lösemittel umsetzt,
oder daß man

[F] das Enamin der Formel (IV) mit Ylidenverbindungen der allgemeinen Formel (VII) in Gegenwart inerter Lösemittel umsetzt,
oder daß man

[G] 1,4-Dihydropyridincarbonsäuren der allgemeinen Formel (X)

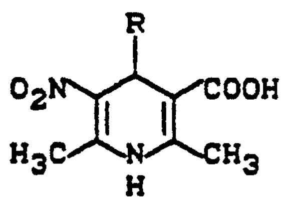 (X)

in welcher
R die angegebene Bedeutung hat,
nach üblichen Methoden der Veresterung von Carbonsäuren (z.B. über das Säurechlorid oder -imidazolid oder in Gegenwart von Dicyclohexylcarbodiimid) mit dem Alkohol der Formel (XI)

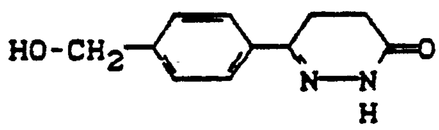

(XI)

verestert.

5. Verfahren gemäß Anspruch 4, dadurch gekennzeichnet, daß man die Umsetzung bei Temperaturen zwischen 20 und 200°C durchführt, wobei bei den Verfahrensvarianten A - F Wasser und inerte organische Lösungsmittel eingesetzt werden und bei den Verfahrensvarianten C und D Ammoniak im Überschuß eingesetzt wird und bei der Verfahrensvariante G Dicarbonsäure zunächst in eine aktivierte Form überführt wird.

6. Verbindungen der allgemeinen Formel (I) gemäß Anspruch 1 zur Verwendung bei der Bekämpfung von Herz-und Kreislauferkrankungen, zur Senkung des Blutzuckers, zur Abschwellung von Schleimhäuten und zur Beeinflussung des Salz-und Flüssigkeitshaushaltes.

7. Arzneimittel enthalten mindestens eine Verbindung der allgemeinen Formel (I) gemäß Anspruch 1.

8. Verfahren zur Herstellung von Arzneimitteln, dadurch gekennzeichnet, daß man Verbindungen der allgemeinen Formel (I) gemäß Anspruch 1 gegebenenfalls unter Verwendung von Hilfs-und Trägerstoffen in eine geeignete Applikationsform überführt.

9. Verwendung von Verbindungen der allgemeinen Formel (I) gemäß Anspruch 1 bei der Herstellung von Arzneimitteln zur Behandlung von Herz-und Kreislauferkrankungen, zur Senkung des Blutzuckers, zur Abschwellung von Schleimhäuten und zur Beeinflussung des Salz-und Flüssigkeitshaushaltes.

10. Verwendung von Verbindungen der allgemeinen Formel (I) gemäß Anspruch 1 bei der Bekämpfung von Erkrankungen des Herz-und Kreislaufsystems, zur Senkung des Blutzuckers, zur Abschwellung von Schleimhäuten und zur Beeinflussung des Salz-und Flüssigkeitshaushaltes.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 88105149.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 445 852 (BAYER)<br>* Formel I; Seite 24, Zeilen 1-5 *<br>-- | 1,7 | C 07 D 401/12<br>C 07 D 401/14<br>C 07 D 409/14<br>A 61 K 31/505 |
| A | DE - A1 - 3 431 862 (BAYER)<br>* Formel I; Beispiel 8 *<br>-- | 1 | |
| A | DE - A1 - 3 311 005 (BAYER)<br>* Formel I; Beispiele 2,8; Anspruch 6 *<br>-- | 1,7 | |
| A | DE - A1 - 3 339 861 (BAYER)<br>* Formel I *<br>---- | 1 | |

RECHERCHIERTE SACHGEBIETE (Int. Cl.4)

C 07 D 401/00
C 07 D 409/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9
Unvollständig recherchierte Patentansprüche: -
Nicht recherchierte Patentansprüche: 10
Grund für die Beschränkung der Recherche:

(Artikel 52(4) EPÜ; Verfahren zur therapeutischen Behandlung des menschlichen oder tierischen Körpers)

| Recherchenort | Abschlußdatum der Recherche | Prüfer |
|---|---|---|
| WIEN | 06-06-1988 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 08.82